# FASCICULE DE BREVET EUROPEEN

(11) **EP 1 302 194 B1**
(45) Date de publication et mention de la délivrance du brevet: **05.07.2006**
(21) Numéro de dépôt: 02292446.8
(22) Date de dépôt: 04.10.2002
(51) Int. Cl.: A61Q 5/04, A61K 8/43

(54) **Utilisation d'un composé de polyguanidine pour le traitement ou la mise en forme des cheveux, notamment le défrisage ou la permanente**
Verwendung von einem Polyguanidinverbindung zur Behandlung oder Verformung des Haares, insbesondere zum Wellen und Glätten
Use of a polyguanidine compound for treating or styling hair, especially for straightening or permanent waving

(30) Priorité: 09.10.2001 FR 0112973
(43) Date de publication de la demande: 16.04.2003
(73) Titulaire: L'ORÉAL, 75008 Paris (FR)
(72) Inventeur: Philippe, Michel, 91320 Wissous (FR)
(74) Mandataire: Casalonga, Axel

(56) Documents cités:
- EP-A- 0 439 698
- WO-A-99/54291
- FR-A- 2 514 640
- US-A- 5 932 201
- DATABASE EPODOC [en ligne] EUROPEAN PATENT OFFICE, THE HAGUE, NL; XP002230463 & RU 2 106 859 A (N.K. SENJAVINA ET AL.) 20 mars 1998 (1998-03-20)

## Description

La présente invention se rapporte à l'utilisation de polyguanidines ou de leurs sels physiologiquement acceptables pour la protection des fibres kératiniques lors de traitements de mise en forme permanente ou d'opération de défrisage.

L'invention se rapporte encore à un procédé de traitement cosmétique de mise en forme permanente ou de défrisage des fibres kératiniques tel qu'il consiste à appliquer sur les fibres kératiniques une composition contenant un composé dérivé de polyguanidines.

Les polyalkylèneguanidines sont les copolymères obtenus par condensation d'hydrochlorure de guanidine avec une alkylènediamine. De tels composés et leurs préparations à partir d'hexaméthylène diamine sont connus depuis 1975 par Zh. Prikl. Khim. (Léningrad) (1975), 48 (8), 1833-6, de GEMBITSKII, P., A. et al.

Les polyalkylèneguanidines sont connues pour leur activité antibactérienne, cette utilisation est notamment décrite dans les brevets RU 2 143 905 et SU 1 687 261. Plus récemment, l'utilisation des polyalkylèneguanidines pour le soin de la peau a été décrite. Ainsi, les brevets SU 1 803 099 et RU 2 106 859 décrivent les propriétés cicatrisantes et émollientes de ces composés.

La demande de brevet SU 857 257 décrit les propriétés hautement détergentes de compositions à base de polyhexaméthylène-guanidines.

On connaît différents procédés de synthèse de ces dérivés polyalkylèneguanidines.

Les demandes de brevets EP 0 439 698, WO 99/54291, ou encore RU 2 052 453 présentent des alternatives aux procédés de synthèse des polyalkylèneguanidines décrits dans la publication de GEMBITSKII et al., citée ci-dessus.

De façon avantageuse et surprenante, la Demanderesse a découvert que des composés dérivés de polyalkylèneguanidines de formule (I) que l'on définira ci-dessous, pouvaient être utilisés en cosmétique pour le soin des fibres kératiniques lors des traitements de mise en forme permanente.

De préférence, le composé dérivé de polyguanidines entrera dans la composition réductrice utilisée lors de la mise en forme de permanentes.

Il est connu que les cheveux régulièrement soumis à des opérations de permanentes perdent leurs propriétés mécaniques. Ces cheveux permanentés sont plus cassants, ils sont difficiles à mettre en forme, de plus il est difficile d'obtenir une coloration uniforme sur des cheveux permanentés.

Il a également été remarqué que les traitements cosmétiques visant à défriser les matières kératiniques et en particulier les cheveux, ont également pour conséquence d'altérer les propriétés mécaniques desdites matières kératiniques.

La demanderesse a ainsi montré que l'utilisation de compositions contenant des composés dérivés de polyguanidines lors de la mise en forme de permanentes ou d'opération de défrisage des cheveux permet de limiter voire d'annuler les effets dégradants des permanentes.

Les composés dérivés de polyguanidine utilisables dans le cadre de la présente invention répondent à la formule (I) suivante: 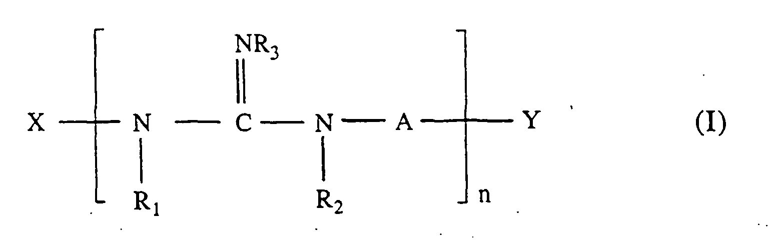 dans laquelle :
X, R₁, R₂, R₃, indépendamment l'un de l'autre, sont choisis dans le groupe formé par un atome d'hydrogène, un radical hydroxyle, un radical alkyle en C₁ à C₁₆, de préférence en C₁ à C₈, linéaire ou ramifié, saturé ou insaturé, hydroxylé ou non,
Y désigne un radical NHR, dans lequel R est choisi dans le groupe formé par un atome d'hydrogène, un radical hydroxyle, un radical alkyle en C₁ à C₁₆, de préférence en C₁ à C₈, linéaire ou ramifié, saturé ou insaturé, hydroxylé ou non,
A désigne un radical alkylène en C₁ à C₁₆, de préférence en C₁ à C₁₂, linéaire ou ramifié, saturé ou insaturé, substitué ou non par au moins un radical choisi parmi un radical hydroxyle, carboxyle (-COOH), carboxylate, un halogène, ledit radical alkylène pouvant contenir au moins une fonction amine (-NH-), éther (-O-), thioéther (-S-), ester (-(CO)O- ou -O(CO)-), amide (-CONR- ou -NRCO- où R est hydrogène, un radical alkyle en C₁ à C₈), carbamate (-NH(CO)O- ou - O(CO)NH-), urée (-NH(CO)NH-), un cycle arylique en C₆ ou cyclanique en C₃ à C₈ éventuellement substitué par un alkyle en C₁-C₈, hydroxyle, halogène; ou A désigne un ou plusieurs cycles aryliques en C₆ ou C₇ ou cyclaniques en C₅ à C₇ éventuellement substitués par un alkyle en C₁-C₈, un groupe hydroxyle ou un halogène ou non substitués ; ou A désigne une chaîne polyarylique en C₁₀ à C₁₄ ou polycyclanique en C₆ à C₁₀, pouvant être interrompue par au moins un radical alkylène en C₁ à C₈, une fonction amine (-NH-), amide (-CONR- ou NRCO où R est un radical alkyle en C₁ à C₈), éther (-O-), thioéther (-S-), un atome d'hydrogène, carbamate (-NH(CO)O- ou - O(CO)NH-), urée (-NH(CO)NH-) ;
n est un entier compris entre 2 et 5000, et de préférence entre 2 et 200, et ses sels physiologiquement acceptables.

Par « carboxylate », au sens de la présente demande, on entend un sel d'addition d'un acide carboxylique avec une base notamment choisie parmi la soude, la potasse, l'ammoniaque, les amines ou les alcanolamines ou bien un sel interne ampholyte avec un groupement guanidinium de la chaîne.

Par « cycle arylique en C₆», au sens de la présente demande, on entend un noyau benzénique, ce noyau pouvant être substitué par un ou deux radicaux alkyles en C₁ ou C₈, OH, halogène.

Par « chaîne polyarylique en C₁₀ à C₁₄ », au sens de la présente demande, on entend une chaîne contenant 2, 3 noyaux aromatiques, chacun étant éventuellement substitué par un ou deux radicaux alkyles en C₁ à C₈, OH, halogène.

De préférence, il s'agira plus particulièrement des dérivés de formule (I) définie ci-dessus, dans laquelle A désigne un radical alkylène en C₁ à C₆, linéaire ou ramifié, saturé ou insaturé, substitué ou non par au moins un radical choisi parmi les radicaux hydroxyle, carboxyle, carboxylate ou les halogènes (le fluor, le chlore, le brome, l'iode).

En tant que composés dérivés de polyguanidines, on pourra utiliser des homopolymères aussi bien que des hétéropolymères.

Par "homopolymère" au sens de la présente demande, on entend une chaîne polymérique constituée de motifs (-N(R₁)-C(NR₃)-N(R₂)-A), dans lesquels R₁, R₂, R₃ et A sont toujours identiques, et par "hétéropolymère", un polymère pour lequel au moins l'une des unités R₁, R₂, R₃ et A est différente des autres.

De façon encore plus préférée, les composés utilisés dans les compositions pour la protection et/ou le soin des fibres kératiniques au sens de la présente invention, sont les sels de polytétraméthylène guanidinium et de polyhexaméthylèneguanidinium et plus particulièrement les halogénures (fluorure, chlorure et bromure), les carboxylates (gluconates, acétates, lactates) ou bien un sel interne ampholyte avec un groupement guanidinium de la chaîne, de manière préférée il s'agira du chlorure de polyhexaméthylèneguanidinium, du chlorure de polytétraméthylèneguanidinium et de l'acétate de polytétraméthylèneguanidinium.

Les dérivés de polyguanidine utilisables dans le cadre de la présente invention peuvent être préparés en mettant en oeuvre tout procédé de préparation connu de l'art antérieur. En particulier, les composés dérivés de polyguanidine selon la présente invention, sont préparés en mélangeant une alkylènediamine et un sel de guanidine, par exemple l'hydrochlorure de guanidine selon un rapport environ équimolaire, puis en chauffant ce mélange à une température comprise entre 120 et 150°C pendant une durée comprise entre 4 et 10 heures. Le mélange de l'alkylènediamine et du sel de guanidine pourra être effectué en masse ou en présence d'un solvant, qui sera de préférence le polyéthylèneglycol (PEG).

Le PEG présente l'avantage d'être un bon solvant pour les réactifs : alkylènediamine et sel de guanidine, par contre le composé dérivé de polyguanidine obtenu à l'issu de la réaction n'est pas miscible avec le PEG. Ce procédé permet donc de récupérer directement le composé dérivé de polyguanidine et aussi de récupérer le PEG qui pourra être réutilisé en tant que solvant, ainsi que les réactifs en excès ou qui n'auront pas eu le temps de réagir.

Selon une autre variante du procédé de préparation des composés dérivés de polyguanidines selon l'invention, on peut effectuer l'étape de chauffage en deux temps : une première étape de chauffage à une température comprise entre 80 et 120°C, et de préférence d'environ 120°C, pendant une période comprise entre 4 et 5 heures, puis une deuxième étape de chauffage à une température comprise entre 120 et 160°C et de préférence d'environ 150°C pendant une période comprise entre 8 et 11 heures.

Le procédé de GEMBITSKII et al., cité ci-dessus, ainsi que les procédés décrits dans les demandes de brevets WO 99/54291et EP 439 698 pourront être utilisés pour préparer les composés dérivés de polyguanidines selon la présente invention.

La présente demande a pour objet une composition cosmétique comprenant une association d'au moins un dérivé de polyguanidine de formule (I) ou l'un de ses sels physiologiquement acceptables et d'un composé choisi parmi les composés réducteurs de la kératine et les composés fixateurs de la kératine.

La présente demande concerne encore l'utilisation de cette composition cosmétique pour mettre en oeuvre une mise en forme permanente ou un défrisage, il pourra s'agir d'une composition réductrice ou d'une composition fixatrice, de préférence oxydante.

La présente demande concerne encore un dispositif à plusieurs compartiments, ou "kit" tel que l'un des compartiments comprend une composition réductrice contenant une association d'un composé dérivé de polyguanidine et d'un composé réducteur de la kératine et un dispositif à plusieurs compartiments, ou "kit" tel que l'un des compartiments comprend une composition fixatrice contenant une association d'un composé dérivé de polyguanidine et d'un composé fixateur de la kératine.

Un autre objet de la présente invention comprend un procédé de traitement cosmétique de mise en forme permanente ou de défrisage des fibres kératiniques, en particulier des cheveux, tels qu'il consiste à appliquer sur ces fibres, une composition cosmétique comprenant l'association d'un composé dérivé de polyguanidine ou l'un de ses sels physiologiquement acceptables et d'un composé choisi parmi les composés réducteurs de la kératine et les composés fixateurs de la kératine.

Ledit dérivé de polyguanidine peut être utilisé en association avec tous les composés réducteurs de la kératine ou avec tous les composés fixateurs de la kératine classiques dans toutes compositions cosmétiques classiques pour mettre en oeuvre une permanente ou un défrisage.

De préférence, les composés dérivés de polyguanidine de formule (I) seront utilisés pour ou dans des compositions réductrices pour la mise en oeuvre de permanentes ou pour la mise en oeuvre d'un défrisage.

Les compositions réductrices pour la mise en oeuvre de permanentes peuvent consister en toute composition déjà connue en soi comme composition réductrice.

Plus particulièrement, les compositions réductrices utilisables pour la mise en oeuvre de permanentes contiennent, à titre d'agents réducteurs de la kératine, des sulfites et/ou des bisulfites, notamment d'alcalins, d'alcalino-terreux ou d'ammonium ou, de préférence, des thiols. Parmi ces derniers, ceux les plus couramment utilisés sont la cystéïne et ses divers dérivés (notamment la N-acétylcystéïne), la cystéamine et ses divers dérivés (notamment ses dérivés acylés en C₁₋C₄ tels que la N-acétyl cystéamine ou la N-propionyl cystéamine), l'acide thiolactique et ses esters (notamment le monothiolactate de glycérol), l'acide thioglycolique ainsi que ses esters, notamment le monothioglycolate de glycérol ou de glycol, et le thioglycérol. On peut également mentionner les réducteurs, suivants: les N-mercapto-alkylamides de sucres tels que le N-(mercapto-2-éthyl)gluconamide, l'acide β-mercaptopropionique et ses dérivés, l'acide thiomalique, la panthétéïne, les N-(mercaptoalkyl)ω-hydroxyalkylamides décrits dans la demande de brevet EP-A-354 835 et les N-mono- ou N,N-dialkylmercapto 4-butyramides décrits dans la demande de brevet EP-A-368 763, les aminomercaptoalkylamides décrits dans la demande de brevet EP-A-432 000 et les alkylaminomercaptoalkylamides décrits dans la demande de brevet EP-A-514 282, le mélange de thioglycolate d'hydroxy-2 propyle (2/3) et de thioglycolate d'hydroxy-2 méthyl-1 éthyle (67/33) décrit dans la demande de brevet FR-A-2 679 448.

Ces agents réducteurs de la kératine sont généralement mis en oeuvre dans des compositions cosmétiquement acceptables, lesquelles sont par ailleurs déjà bien connues en soi dans l'état de l'art existant des formulations frisantes destinées à réaliser la première étape (réduction) d'une opération de permanente. Ainsi, à titre d'additifs usuels et classiques, utilisables seuls ou en mélanges, on peut plus particulièrement mentionner les agents tensio-actifs de type non-ionique, anionique, cationique ou amphotère et parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés, ainsi que d'autres tensio-actifs non-ioniques du type hydroxypropyléther.

Lorsque la composition réductrice contient au moins un agent tensio-actif, celui-ci est généralement présent à une concentration maximale de 30% en poids, et de préférence comprise entre 0,5 et 10% en poids, par rapport au poids total de la composition réductrice.

Dans le but d'améliorer les propriétés cosmétiques des cheveux ou encore d'en atténuer ou d'éviter leur dégradation, la composition réductrice peut également, en plus du composé dérivé de polyguanidine, contenir un agent traitant de nature cationique, anionique, non-ionique ou amphotère.

Parmi les agents traitants particulièrement préférés, on peut notamment citer ceux décrits dans les demandes de brevets français n° 2 598 613 et 2 470 596. On peut également utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2 535 730, les polyorganosiloxanes à groupements aminoalkyles modifiés par des groupements alcoxycarbonylalkyles tels que ceux décrits dans le brevet US n° 4 749 732, des polyorganosiloxanes tels que le copolymère polydiméthylsiloxane-polyoxyalkyle du type Diméthicone Copolyol, un polydiméthylsiloxane à groupements terminaux stéaroxy-(stéaroxydiméthicone), un copolymère polydiméthyl-siloxanedialkylammonium acétate ou un copolymère polydiméthyl-siloxane polyalkylbétaïne décrits dans la demande de brevet britannique n° 2 197 352, des polysiloxanes organo modifiés par des groupements mercapto ou mercaptoalkyles tels que ceux décrits dans le brevet français n° 1 530 369 et dans la demande de brevet européen n° 295 780, ainsi que des silanes tels que le stéaroxytriméthylsilane.

La composition réductrice peut également contenir d'autres ingrédients traitants tels que des polymères cationiques tels que ceux utilisés dans les compositions de brevets français n° 79 32078 (FR-A-2 472 382) et 80 26421 (FR-A-2 495 931), ou encore des polymères cationiques du type ionène tels que ceux utilisés dans les compositions du brevet luxembourgeois n° 83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration ou permettant de renforcer l'efficacité du réducteur tels que le mélange SiO₂/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylène-glycol tels que par exemple le monométhyléther de propylèneglycol, le monométhyléther de dipropylèneglycol, le monoéthyléther de l'éthylèneglycol et le monoéthyléther du diéthylèneglycol, des alcanediols en C₃-C₆ tels que par exemple le propanediol-1,2 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs.

Dans des compositions réductrices de permanente, les agents réducteurs tels que ceux mentionnés ci-avant sont généralement présents à une concentration qui peut être comprise entre 1 et 30% en poids, et de préférence entre 5 et 20% en poids par rapport au poids total de la composition réductrice.

La composition réductrice peut se présenter sous la forme d'une lotion, épaissie ou non, d'une crème, d'un gel, ou de toute autre forme appropriée.

La composition réductrice peut être également du type exothermique, c'est-à-dire provoquant un certain échauffement lors de l'application sur les cheveux, ce qui apporte un agrément à la personne qui subit la permanente ou le défrisage.

La composition réductrice peut également contenir un solvant tel que par exemple de l'éthanol, du propanol ou de l'isopropanol, ou encore du glycérol à une concentration maximale de 20% par rapport au poids total de la composition.

Le véhicule des compositions est de préférence l'eau ou une solution hydroalcoolique d'un alcool inférieur tel que l'éthanol, l'isopropanol ou le butanol.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes sous forme d'émulsions « lourdes ».'

Par exemple, pour obtenir une crème, on peut émulsionner une phase aqueuse contenant en solution le composé dérivé de polyguanidines et éventuellement d'autres ingrédients ou adjuvants, et une phase huileuse.

La phase huileuse peut être constituée par divers produits tels que l'huile de paraffine, l'huile de vaseline, l'huile d'amande douce, l'huile d'avocat, l'huile d'olive, des esters d'acides gras comme le monostéarate de glycéryle, les palmitates d'éthyl ou d'isopropyle, les myristates d'alkyle tels que les myristates de propyle, de butyle ou de cétyle. On peut en outre ajouter des alcools gras comme l'alcool cétylique ou des cires telles que par exemple la cire d'abeille.

On peut également utiliser des liquides ou des gels contenant des agents épaississants tels que des polymères ou des copolymères carboxyvinyliques qui « collent » les cheveux et les maintiennent dans la position lisse pendant le temps de pose.

Enfin, les compositions réductrices peuvent également contenir au moins un disulfure connu pour son utilisation dans une composition réductrice pour permanente auto-neutralisante.

Parmi de tels disulfures connus, on peut notamment mentionner l'acide dithioglycolique, le dithioglycérol, la cystamine, la N,N'-diacétyl-cystamine, la cystine, la pantéthine, et les disulfures des N-(mercapto-alkyl)ω-hydroxyalkylamides décrits dans la demande de brevet européen EP 354 835, les disulfures des N-mono ou N,N-dialkylmercapto-4-butyramides décrits dans la demande de brevet EP 368 763, les disulfures des aminomercapto-alkylamides décrits dans la demande de brevet EP 432 000, et les disulfures des alkylaminomercaptoalkylamides décrits dans la demande de brevet EP 514 282. Ces disulfures sont généralement présents dans un rapport molaire de 0,5 à 2,5, et de préférence de 1 à 2, par rapport à l'agent réducteur (voir brevet US 3 768 490).

Les pH des compositions réductrices peuvent être ajustés classiquement par ajout, soit d'agents basifiants, tels que par exemple l'ammoniaque, la monoéthanolamine, la diéthanolamine, la triéthanolamine, l'isopropanolamine, la propanediamine-1,3, un carbonate ou bicarbonate alcalin ou d'ammonium, un carbonate organique tel que le carbonate de guanidine (compositions réductrices carbonatées) ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique ou l'acide borique.

Les compositions comprenant, en association, au moins un dérivé de polyguanidine de formule (I) et au moins un composé réducteur choisi parmi les sulfites ou les bisulfites tels que définis ci-dessus, peuvent être appliquées de manière répétée sur les cheveux pour la mise en oeuvre de permanentes ou de défrisages sans que l'on observe une modification importante du comportement de ces cheveux, en particulier au niveau de leur aptitude à être par la suite correctement colorés.

En effet, on observe généralement que, sur les cheveux qui ont subi quelques opérations de permanente ou de défrisage (de l'ordre de trois au maximum), la coloration sera beaucoup plus prononcée que celle obtenue sur les mêmes cheveux, mais non permanentés. Ceci pose donc un problème dans tous les cas où l'opération de coloration est conduite sur une chevelure à l'origine permanentée mais qui, entre temps, a repoussé (mauvaise unisson entre les cheveux d'origine permanentés et les cheveux de repousse non permanentés).

On observe également que la coloration devient très difficile, voire impossible, si la chevelure à colorer a subi auparavant de nombreuses opérations de permanentes ou de défrisages, en particulier plus de cinq permanentes.

Il convient de remarquer que les compositions cosmétiques utilisées dans le cadre de l'invention sont aussi bien des compositions prêtes à l'emploi que des concentrés devant être dilués avant l'utilisation. Les compositions cosmétiques ne sont donc pas limitées à un domaine particulier de concentration des composés dérivés de polyguanidines.

Généralement, dans les compositions cosmétiques utilisées, la concentration en composés de dérivés de polyguanidines est comprise entre 0,001 et 25% en poids, et de préférence, entre 0,1 et 10% en poids par rapport au poids total de la composition.

### EXEMPLES

### I Effet protecteur de la fibre

Dans le but d'évaluer leurs propriétés de protection des fibres kératiniques, des compositions contenant un dérivé de polyguanidine ont été soumises à un test dit « de gonflement diamétral dans un liquide ».

Sans vouloir être lié à aucune théorie, ce test est basé sur le principe suivant : le diamètre du cheveu augmente dans l'eau, ce gonflement dépend entre autre de la sensibilité du cheveu. L'augmentation se stabilise après quelques minutes. Ainsi, plus le cheveu sera sensibilisé, c'est-à-dire dégradé, plus son diamètre, lorsqu'il est placé dans un liquide, augmente.

Il a en outre été observé un gonflement supplémentaire dans la phase de réduction lors d'une opération de permanente. Sans être lié à aucune théorie, ce gonflement semble être la conséquence de la rupture des ponts disulfures et de la génération des thiolates. Ce gonflement se stabilise environ 8 minutes après le début de la phase de réduction.

Il a également été observé un surgonflement supplémentaire lorsque la fibre capillaire est rincée au moyen d'eau permutée. Sans être lié à aucune théorie, ce surgonflement semble être dû au choc osmique, à la répulsion électrostatique entre les chaînes des fibres capillaires.

Les mesures suivantes ont été réalisées à l'aide d'un système de mesure optique Zimmer, sans contact. Selon ce système, un cheveu est suspendu dans une cuve en quartz, dans laquelle les différents liquides de traitement sont successivement introduits. L'évolution du diamètre de la fibre est enregistrée en continu au cours des différentes phases du traitement. Les résultats sont obtenus à partir de quatre à sept essais pour chaque traitement réalisé.

Le test de gonflement en milieu de permanente est notamment décrit dans les publications suivantes :
- J. Nothen « Proceedings » 16è Congrès IFSCC 1990, page 315.
- A. Schansky « Journal of Society of Cosmetic Chemist » Vol. 14, 1963, page 427.
- K.W. Herrmann « Transaction Faraday Society» Vol.59 p.1663,année 1963.

### Exemples comparatifs.

Des cheveux décolorés sont traités séparément au moyen de trois solutions réductrices, Red₁, Red₂ et Red₃, puis rincés. L'évolution du gonflement diamétral est mesurée en phase de réduction et lors du rinçage. Le déroulement de ces tests ainsi que les résultats sont présentés ci-dessous.

Le surgonflement correspond à la différence entre le début de gonflement lors du rinçage (à T+13) et la fin de gonflement dans le réducteur (à T + 11).

Red₁ est une solution d'acide thioglycolique (TGA) à 7% en poids amenée à pH 8 au moyen d'ammoniaque.

Red₂ est une solution d'acide thioglycolique à 7% en poids amenée à pH 8 au moyen d'ammoniaque et comprenant 1 g de chlorure de polyhexaméthylèneguanidinium.

Red₃ est une solution d'acide glycolique à 7% en poids amenée à pH 8 au moyen d'ammoniaque et comprenant 1% en poids de chlorure de polytétraméthylèneguanidinium.

Pour le test, les traitements suivants sont successivement effectués sur le cheveu :
- eau distillée pendant 2 minutes (T + 2)
- solution réductrice pendant 9 minutes (T + 11)
- eau distillée pendant 2 minutes (T + 13)
- eau distillée pendant 2 minutes (T + 15).

| Phase de réduction | Fin de gonflement dans le réducteur (%) à T+11 | Début de gonflement lors du rinçage (%) à T+13 | Fin de gonflement lors du rinçage (%) à T+15 | Surgonflement (%) |
|---|---|---|---|---|
| Red₁ | 22,9 | 32,6 | 35,8 | 9,7 |
| Red₂ | 23,15 | 26,28 | 16,33 | 3,15 |
| Red₃ | 14,84 | 10,46 | 6,6 | -4,37 |

Ce test simule l'augmentation du surgonflement des fibres kératiniques lors d'une opération de permanente.

Ce test montre l'efficacité du chlorure de polytétraméthylène guanidinium et du chlorure de polyhexaméthylène-guanidinium en tant qu'agents protecteurs des fibres dans des compositions réductrices utilisables lors d'opérations de permanentes.

La composition à base de chlorure de polytétraméthylèneguanidine permet même une diminution du surgonflement de la fibre généralement observée dans la phase de réduction d'une opération de permanente.

### II Exemples de compositions permanentes

**Composition réductrice 1 (en poids)**

| | |
|---|---|
| Chlorure de polyhexaméthylèneguanidium | 1% |
| Acide thioglycolique | 8% |
| Monoéthanolamine | qs pH 7 |
| Eau | qsp 100 |

**Composition réductrice 2 (en poids)**

| | |
|---|---|
| Chlorure de polyhexaméthylèneguanidium | 5% |
| Monothioglycolate de glycérol en solution à 68% en poids de glycérol | 8% |
| Monoéthanolamine | qs pH 7 |
| Eau | qsp 100 |

## Revendications

1. Composition cosmétique comprenant l'association d'un composé dérivé de polyguanidine de formule (I) : 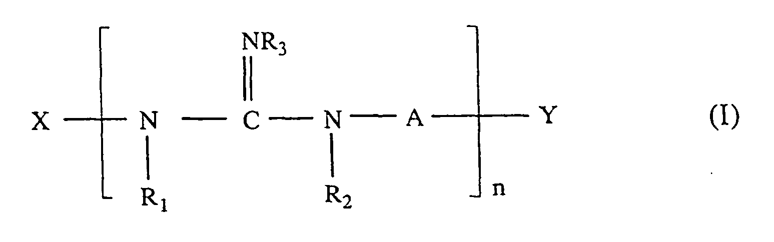 dans laquelle :
X, R₁, R₂, R₃, indépendamment l'un de l'autre, sont choisis dans le groupe formé par un atome d'hydrogène, un radical hydroxyle, un radical alkyle en C₁ à C₁₆, de préférence en C₁ à C₈, linéaire ou ramifié, saturé ou insaturé, hydroxylé ou non,
Y désigne un radical NHR, dans lequel R est choisi dans le groupe formé par un atome d'hydrogène, un radical hydroxyle, un radical alkyle en C₁ à C₁₆, de préférence en C₁ à C₈, linéaire ou ramifié, saturé ou insaturé, hydroxylé ou non,
A désigne un radical alkylène en C₁ à C₁₆, de préférence en C₁ à C₁₂, linéaire ou ramifié, saturé ou insaturé, substitué ou non par au moins un radical choisi parmi un radical hydroxyle, carboxyle (-COOH), carboxylate, un halogène, ledit radical alkylène pouvant contenir au moins une fonction amine (-NH-), éther (-O-);thioéth.er (-S-), ester (-(CO)O- ou -O(CO)-), amide (-CONR- ou -NRCO- où R est hydrogène, un radical alkyle en C₁ à C₈), carbamate ( -NH(CO)O- ou -O(CO)NH- ), urée (-NH(CO)NH-), un cycle arylique en C₆ ou cyclanique en C₃ à C₈ éventuellement substitué par un alkyle en C₁-C₈, hydroxyle, halogène; ou A désigne un ou plusieurs cycles aryliques en C₆ ou C₇ ou cyclaniques en C₅ à C₇, substitués par un alkyle en C₁-C₈, un groupe hydroxyle ou un halogène ou non substitués ; ou A désigne une chaîne polyarylique en C₁₀ à C₁₄ ou polycyclanique en C₆ à C₁₀, pouvant être interrompue par au moins un radical alkylène en C₁ à C₈, une fonction amine (-NH-), amide (-CONR- ou NRCO où R est un radical alkyle en C₁ à C₈), éther (-O-), thioéther (-S-) un atome d'hydrogène, carbamate (-NH(CO)O- ou -O(CO)NH-), urée (-NH(CO)NH-);
n est un entier compris entre 2 et 5000, et de préférence entre 2 et 200, ou un de ses sels physiologiquement acceptables
et d'un composé choisi parmi les composés réducteurs de la kératine et les composés fixateurs de la kératine.

2. Composition cosmétique selon la revendication 1 tel que le composé dérivé de polyguanidine est choisi dans le groupe formé par les sels de polytétraméthylèneguanidinium et de polyhexaméthylèneguanidinium.

3. Composition cosmétique selon la revendication 2 tel que les sels de polytétraméthylèneguanidinium et de polyhexaméthylène-guanidinium sont choisis dans le groupe formé par les halogénures (fluorure, chlorure et bromure), les carboxylates (gluconate, acétate, lactate), les sels internes ampholytes avec un groupement guanidinium de la chaîne.

4. Composition cosmétique selon la revendication 3 telle que le composé dérivé de polyguanidine est choisi dans le groupe formé par le chlorure de polyhexaméthylèneguanidinium, le chlorure de polytétraméthylèneguanidinium et l'acétate de polytétraméthylèneguanidinium.

5. Composition cosmétique selon l'une des revendications précédentes telle que le composé réducteur de la kératine est choisi parmi les sulfites ou les bisulfites.

6. Composition cosmétique selon la revendication 5 telle que le composé réducteur est choisi parmi les thiols et en particulier la cystéine et ses dérivés, la cystéamine et ses dérivés, l'acide thiolactique et ses esters, l'acide thioglycolique et ses esters.

7. Utilisation d'une composition cosmétique selon l'une des revendications 1 à 6 pour effectuer une mise en forme permanente ou un défrisage des fibres kératiniques.

8. Utilisation d'une composition cosmétique selon la revendication 7 telle que la composition cosmétique est une composition réductrice.

9. Utilisation d'une composition cosmétique selon l'une des revendications 1 à 6 telle que cette composition cosmétique est fixatrice pour effectuer une mise en forme permanente des fibres kératiniques.

10. Utilisation selon l'une des revendications 7 ou 8 telle que le composé de polyguanidine représente de 0,001 à 25% en poids et de préférence de 0,1 à 10% en poids par rapport au poids total de la composition cosmétique.

11. Utilisation selon l'une des revendications 7 ou 8 telle que la composition cosmétique contient au moins un agent choisi parmi les tensioactifs non ioniques, anioniques, cationiques ou amphotères, les agents traitants non ioniques, anioniques, cationiques, ou amphotères.

12. Procédé de traitement de cosmétique de mise en forme permanente ou de défrisage des fibres kératiniques tel qu'il consiste à appliquer sur les fibres kératiniques une composition cosmétique comprenant une association d'un composé dérivé de polyguanidine de formule (I) : 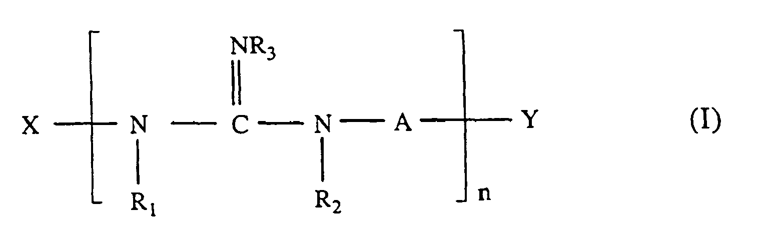 dans laquelle :
X, R₁, R₂, R₃, indépendamment l'un de l'autre, sont choisis dans le groupe formé par un atome d'hydrogène, un radical hydroxyle, un radical alkyle en C₁ à C₁₆, de préférence en C₁ à C₈, linéaire ou ramifié, saturé ou insaturé, hydroxylé ou non,
Y désigne un radical NHR, dans lequel R est choisi dans le groupe formé par un atome d'hydrogène, un radical hydroxyle, un radical alkyle en C₁ à C₁₆, de préférence en C₁ à C₈, linéaire ou ramifié, saturé ou insaturé, hydroxylé ou non,
A désigne un radical alkylène en C₁ à C₁₆, de préférence en C₁ à C₁₂, linéaire ou ramifié, saturé ou insaturé, substitué ou non par au moins un radical choisi parmi un radical hydroxyle, carboxyle (-COOH), carboxylate, un halogène, ledit radical alkylène pouvant contenir au moins une fonction amine (-NH-), éther (-O-), thioéther (-S-), ester (-(CO)O- ou -O(CO)-), amide (-CONR- ou -NRCO- où R est hydrogène, un radical alkyle en C₁ à C₈), carbamate ( -NH(CO)O- ou -O(CO)NH- ), urée (-NH(CO)NH-), un cycle arylique en C₆ ou cyclanique en C₃ à C₈ éventuellement substitué par un alkyle en C₁-C₈, hydroxyle, halogène; ou A désigne un ou plusieurs cycles aryliques en C₆ ou C₇ ou cyclaniques en C₅ à C₇, substitués par un alkyle en C₁-C₈, un groupe hydroxyle ou un halogène ou non substitués ; ou A désigne une chaîne polyarylique en C₁₀ à C₁₄ ou polycyclanique en C₆ à C₁₀, pouvant être interrompue par au moins un radical alkylène en C₁ à C₈, une fonction amine (-NH-), amide (-CONR- ou NRCO où R est un radical alkyle en C₁ à C₈), éther (-O-), thioéther (-S-) un atome d'hydrogène, carbamate (-NH(CO)O- ou -O(CO)NH-), urée (-NH(CO)NH-);
n est un entier compris entre 2 et 5000, et de préférence entre 2 et 200, ou un de ses sels physiologiquement acceptables,
et d'un composé choisi parmi les composés réducteurs de la kératine et les composés fixateurs de la kératine.

13. Dispositif à plusieurs compartiments, ou "kit" **caractérisé en ce que** l'un des compartiments comprend une composition cosmétique réductrice contenant une association d'un composé dérivé de polyguanidine et d'un composé réducteur de la kératine selon l'une des revendications 1 à 6.

14. Dispositif à plusieurs compartiments, ou "kit" **caractérisé en ce que** l'un des compartiments comprend une composition cosmétique fixatrice contenant une association d'un composé dérivé de polyguanidine et d'un composé fixateur de la kératine selon l'une des revendications 1 à 4.

## Claims

1. Cosmetic composition comprising the combination of a compound derived from polyguanidine of formula (I): 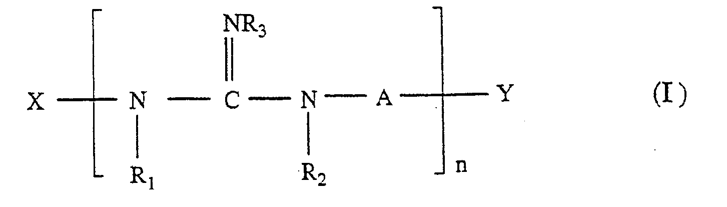 in which:
X, R₁, R₂ and R₃, independently of each other, are chosen from the group formed by a hydrogen atom, a hydroxyl radical and a linear or branched, saturated or unsaturated, optionally hydroxylated C₁ to C₁₆ and preferably C₁ to C₈ alkyl radical,
Y denotes a radical NHR, in which R is chosen from the group formed by a hydrogen atom, a hydroxyl radical and a linear or branched, saturated or unsaturated, optionally hydroxylated C₁ to C₁₆ and preferably C₁ to C₈ alkyl radical,
A denotes a linear or branched, saturated or unsaturated, C₁ to C₁₆ and preferably C₁ to C₁₂ alkylene radical optionally substituted with at least one radical chosen from a hydroxyl, carboxyl (-COOH) or carboxylate radical and a halogen, the said alkylene radical possibly containing at least one amine (-NH-), ether (-O-), thioether (-S-), ester (-(CO)O- or -O(CO)-), amide (-CONR- or -NRCO- in which R is hydrogen or a C₁ to C₈ alkyl radical), carbamate (-NH(CO)O- or -O(CO)NH-) or urea (-NH(CO)NH-) function, a C₆ aryl or C₃ to C₈ cyclanyl ring optionally substituted with a C₁-C₈ alkyl, hydroxyl or halogen; or A denotes one or more C₆ or C₇ aryl or C₅ to C₇ cyclanyl rings substituted with a C₁-C₈ alkyl, a hydroxyl group or a halogen or unsubstituted; or A denotes a C₁₀ to C₁₄ polyaryl or C₆ to C₁₀ polycyclanyl chain, which may be interrupted with at least one C₁ to C₈ alkylene radical, an amine (-NH-), amide (-CONR- or NRCO in which R is a C₁ to C₈ alkyl radical), ether (-O-), thioether (-S-), a hydrogen atom, carbamate (-NH(CO)O- or -O(CO)NH-) or urea (-NH(CO)NH-) function;
n is an integer between 2 and 5 000 and preferably between 2 and 200, or a physiologically acceptable salt thereof,
and of a compound chosen from keratin-reducing compounds and keratin-fixing compounds.

2. Cosmetic composition according to Claim 1, such that the compound derived from polyguanidine is chosen from the group formed by polytetramethyleneguanidinium and polyhexamethyleneguanidinium salts.

3. Cosmetic composition according to Claim 2, such that the polytetramethyleneguanidinium and polyhexamethyleneguanidinium salts are chosen from the group formed by halides (fluoride, chloride and bromide), carboxylates (gluconate, acetate and lactate) and ampholytic internal salts with a guanidinium group of the chain.

4. Cosmetic composition according to Claim 3, such that the compound derived from polyguanidine is chosen from the group formed by polyhexamethyleneguanidinium chloride, polytetramethyleneguanidinium chloride and polytetramethyleneguanidinium acetate.

5. Cosmetic composition according to one of the preceding claims, such that the keratin-reducing compound is chosen from sulphites and bisulphites.

6. Cosmetic composition according to Claim 5, such that the reducing compound is chosen from thiols and in particular cysteine and its derivatives, cysteamine and its derivatives, thiolactic acid and its esters, and thioglycolic acid and its esters.

7. Use of a cosmetic composition according to one of Claims 1 to 6 for carrying out a permanent-shaping or a straightening of keratin fibres.

8. Use of a cosmetic composition according to Claim 7, such that the cosmetic composition is a reducing composition.

9. Use of a cosmetic composition according to one of Claims 1 to 6, such that this cosmetic composition is fixing, for carrying out a permanent-shaping of keratin fibres.

10. Use according to either of Claims 7 and 8, such that the polyguanidine compound represents from 0.001% to 25% by weight and preferably from 0.1% to 10% by weight relative to the total weight of the cosmetic composition.

11. Use according to either of Claims 7 and 8, such that the cosmetic composition contains at least one agent chosen from nonionic, anionic, cationic and amphoteric surfactants, and nonionic, anionic, cationic and amphoteric treating agents.

12. Cosmetic treatment process for permanently shaping or straightening keratin fibres, such that it consists in applying to the keratin fibres a cosmetic composition comprising a combination of a compound derived from polyguanidine of formula (I): 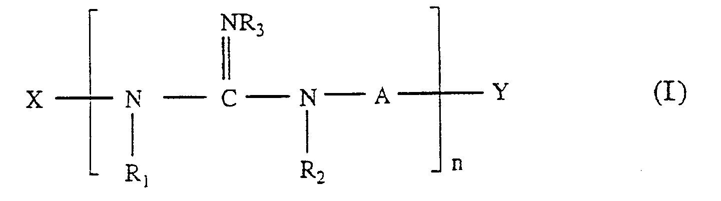 in which:
X, R₁, R₂ and R₃, independently of each other, are chosen from the group formed by a hydrogen atom, a hydroxyl radical and a linear or branched, saturated or unsaturated, optionally hydroxylated C₁ to C₁₋₆ and preferably C₁ to C₈ alkyl radical,
Y denotes a radical NHR, in which R is chosen from the group formed by a hydrogen atom, a hydroxyl radical and a linear or branched, saturated or unsaturated, optionally hydroxylated C₁ to C₁₆ and preferably C₁ to C₈ alkyl radical,
A denotes a linear or branched, saturated or unsaturated, C₁ to C₁₆ and preferably C₁ to C₁₂ alkylene radical optionally substituted with at least one radical chosen from a hydroxyl, carboxyl (-COOH) or carboxylate radical and a halogen, the said alkylene radical possibly containing at least one amine (-NH-), ether (-O-), thioether (-S-), ester (-(CO)O- or -O(CO)-), amide (-CONR- or -NRCO- in which R is hydrogen or a C₁ to C₈ alkyl radical), carbamate (-NH(CO)O- or -O(CO)NH-) or urea (-NH(CO)NH-) function, a C₆ aryl or C₃ to C₈ cyclanyl ring optionally substituted with a C₁-C₈ alkyl, hydroxyl or halogen; or A denotes one or more C₆ or C₇ aryl or C₅ to C₇ cyclanyl rings substituted with a C₁-C₈ alkyl, a hydroxyl group or a halogen or unsubstituted; or A denotes a C₁₀ to C₁₄ polyaryl or C₆ to C₁₀ polycyclanyl chain, which may be interrupted with at least one C₁ to C₈ alkylene radical, an amine (-NH-), amide (-CONR- or NRCO in which R is a C₁ to C₈ alkyl radical), ether (-O-), thioether (-S-), a hydrogen atom, carbamate (-NH(CO)O- or -O(CO)NH-) or urea (-NH(CO)NH-) function;
n is an integer between 2 and 5 000 and preferably between 2 and 200, or a physiologically acceptable salt thereof,
and of a compound chosen from keratin-reducing compounds and keratin-fixing compounds.

13. Multi-compartment device or "kit", **characterized in that** one of the compartments comprises a reducing cosmetic composition containing a combination of a compound derived from polyguanidine and a keratin-reducing compound according to one of Claims 1 to 6.

14. Multi-compartment device or "kit", **characterized in that** one of the compartments comprises a fixing cosmetic composition containing a combination of a compound derived from polyguanidine and a keratin-fixing compound according to one of Claims 1 to 4.

## Patentansprüche

1. Kosmetische Zusammensetzung, die eine von einem Polyguanidin der Formel (I) abgeleitete Verbindung: 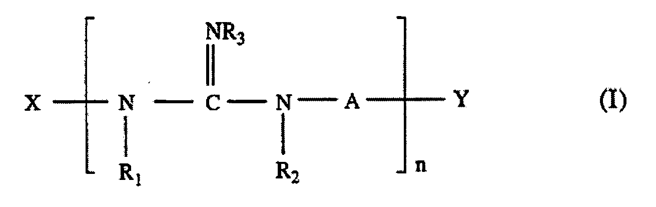 worin bedeuten:
X, R₁, R₂ und R₃ sind unabhängig voneinander unter einem Wasserstoffatom, einer Hydroxygruppe oder einer geradkettigen oder verzweigten, gesättigten oder ungesättigten, hydroxylierten oder nichthydroxylierten C₁₋₁₆-Alkylgruppe und vorzugsweise C₁₋₈-Alkylgruppe ausgewählt,
Y ist eine Gruppe NHR, worin R unter einem Wasserstoffatom, einer Hydroxygruppe oder einer geradkettigen oder verzweigten, gesättigten oder ungesättigten, hydroxylierten oder nichthydroxylierten C₁₋₁₆-Alkylgruppe und vorzugsweise C₁₋₈-Alkylgruppe ausgewählt ist,
A bedeutet eine geradkettige oder verzweigte, gesättigte oder ungesättigte Alkylengruppe mit 1 bis 16 Kohlenstoffatomen und vorzugsweise 1 bis 12 Kohlenstoffatomen, die unsubstituiert vorliegt oder substituiert ist mit mindestens einer Gruppe, die ausgewählt ist unter Hydroxy, Carboxy (-COOH), Carboxylat oder einem Halogen, wobei die Alkylengruppe mindestens eine Aminfunktion (-NH-), Etherfunktion (-O-), Thioetherfunktion (-S-), Esterfunktion (-(CO)O- oder -O(CO)-), Amidfunktion (-CONR- oder -NRCO-, worin R Wasserstoff, C₁₋₈-Alkyl bedeutet), Carbamatfunktion (-NH(CO)O- oder -O(CO)NH-), Harnstofffunktion (-NH(CO)NH-), einen Arylring mit 6 Kohlenstoffatomen oder einen Cyclanring mit 3 bis 8 Kohlenstoffatomen enthalten kann, der gegebenenfalls mit einer C₁₋₈-Alkylgruppe, Hydroxy oder Halogen substituiert ist; oder A bedeutet einen oder mehrere Arylringe mit 6 oder 7 Kohlenstoffatomen oder Cyclanringe mit 5 bis 7 Kohlenstoffatomen, die mit einer C₁₋₈-Alkylgruppe, Hydroxy oder einem Halogen substituiert sind oder unsubstituiert vorliegen; oder A bedeutet eine Polyarylkette mit 10 bis 14 Kohlenstoffatomen oder eine Polycyclankette mit 6 bis 10 Kohlenstoffatomen, die durch mindestens eine Alkylengruppe mit 1 bis 8 Kohlenstoffatomen, eine Aminfunktion (-NH-), eine Amidfunktion (-CONR- oder NRCO, worin R eine C₁₋₈-Alkylgruppe bedeutet), eine Etherfunktion (-O-), eine Thioetherfunktion (-S-), ein Wasserstoffatom, eine Carbamatfunktion (-NH(CO)O)- oder -O(CO)NH-), eine Harnstofffunktion (-NH(CO)NH-) unterbrochen sein kann:
n ist eine ganze Zahl im Bereich von 2 bis 5.000 und vorzugsweise 2 bis 200,
oder ein physiologisch akzeptables Salz dieser Verbindungen, in Kombination mit einer Verbindung enthält, die unter den Verbindungen, die das Keratin reduzieren, und den Verbindungen, die das Keratin fixieren, ausgewählt ist.

2. Kosmetische Zusammensetzung nach Anspruch 1, wobei die von Polyguanidin abgeleitete Verbindung unter den Polytetramethylenguanidiniumsalzen und Polyhexamethylenguanidiniumsalzen ausgewählt ist.

3. Kosmetische Zusammensetzung nach Anspruch 2, wobei die Polytetramethylenguanidiniumsalze und Polyhexamethylenguanidiniumsalze unter den Halogeniden (Fluorid, Chlorid und Bromid), Carboxylaten (Gluconat, Acetat, Lactat) und inneren Ampholytsalzen mit einer Guanidiniumgruppe der Kette ausgewählt sind.

4. Kosmetische Zusammensetzung nach Anspruch 3, wobei die von Polyguanidin abgeleitete Verbindung unter Polyhexamethylenguanidiniumchlorid, Polytetramethylenguanidiniumchlorid und Polytetramethylenguanidiniumacetat ausgewählt ist.

5. Kosmetische Zusammensetzung nach einem der vorhergehenden Ansprüche, wobei die das Keratin reduzierende Verbindung unter den Sulfiten oder Bisulfiten ausgewählt ist.

6. Kosmetische Zusammensetzung nach Anspruch 5, wobei das Reduktionsmittel unter den Thiolen und insbesondere Cystein und seinen Derivaten, Cysteamin und seinen Derivaten, Thiomilchsäure und ihren Estern, Thioglycolsäure und ihren Estern ausgewählt ist.

7. Verwendung einer kosmetischen Zusammensetzung nach einem der Ansprüche 1 bis 6 zur Durchführung einer permanenten Verformung oder Entkräuselung von Keratinfasern.

8. Verwendung einer kosmetischen Zusammensetzung nach Anspruch 7, wobei die kosmetische Zusammensetzung eine reduzierende Zusammensetzung ist.

9. Verwendung einer kosmetischen Zusammensetzung nach einem der Ansprüche 1 bis 6, wobei die kosmetische Zusammensetzung fixierend wirkt, zur Durchführung einer permanenten Verformung von Keratinfasern.

10. Verwendung nach einem der Ansprüche 7 oder 8, wobei die Polyguanidinverbindung 0,001 bis 25 Gew.-% und vorzugsweise 0,1 bis 10 Gew.-%, bezogen auf das Gesamtgewicht der Zusammensetzung, ausmacht.

11. Verwendung nach einem der Ansprüche 7 oder 8, wobei die kosmetische Zusammensetzung mindestens einen Stoff enthält, der unter den nichtionischen grenzflächenaktiven Stoffen, anionischen grenzflächenaktiven Stoffen, kationischen grenzflächenaktiven Stoffen oder amphoteren grenzflächenaktiven Stoffen, nichtionischen, anionischen, kationischen oder amphoteren Behandlungsmitteln ausgewählt ist.

12. Verfahren zur kosmetischen Behandlung für die permanente Verformung oder Entkräuselung von Keratinfasern, das darin besteht, auf die Keratinfasern eine Zusammensetzung aufzutragen, die in Kombination enthält: eine Verbindung, die von einem Polyguanidin der Formel (I) abgeleitet ist: 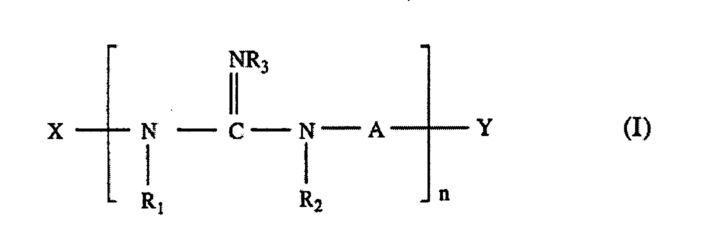 worin bedeuten:
X, R₁, R₂ und R₃ sind unabhängig voneinander unter einem Wasserstoffatom, einer Hydroxygruppe oder einer geradkettigen oder verzweigten, gesättigten oder ungesättigten, hydroxylierten oder nichthydroxylierten C₁₋₁₆-Alkylgruppe und vorzugsweise C₁₋₈-Alkylgruppe ausgewählt,
Y ist eine Gruppe NHR, worin R unter einem Wasserstoffatom, einer Hydroxygruppe oder einer geradkettigen oder verzweigten, gesättigten oder ungesättigten, hydroxylierten oder nichthydroxylierten C₁₋₁₆-Alkylgruppe und vorzugsweise C₁₋₈-Alkylgruppe ausgewählt ist,
A bedeutet eine geradkettige oder verzweigte, gesättigte oder ungesättigte Alkylengruppe mit 1 bis 16 Kohlenstoffatomen und vorzugsweise 1 bis 12 Kohlenstoffatomen, die unsubstituiert vorliegt oder substituiert ist mit mindestens einer Gruppe, die ausgewählt ist unter Hydroxy, Carboxy (-COOH), Carboxylat oder einem Halogen, wobei die Alkylengruppe mindestens eine Aminfunktion (-NH-), Etherfunktion (-O-), Thioetherfunktion (-S-), Esterfunktion (-(CO)O- oder -O(CO)-), Amidfunktion (-CONR-¨ oder -NRCO-, worin R Wasserstoff, C₁₋₈-Alkyl bedeutet), Carbamatfunktion (-NH(CO)O- oder -O(CO)NH-), Harnstofffunktion (-NH(CO)NH-), einen Arylring mit 6 Kohlenstoffatomen oder einen Cyclanring mit 3 bis 8 Kohlenstoffatomen enthalten kann, der gegebenenfalls mit einer C₁₋₈-Alkylgruppe, Hydroxy oder Halogen substituiert ist; oder A bedeutet einen oder mehrere Arylringe mit 6 oder 7 Kohlenstoffatomen oder Cyclanringe mit 5 bis 7 Kohlenstoffatomen, die mit einer C₁₋₈-Alkylgruppe, Hydroxy oder einem Halogen substituiert sind oder unsubstituiert vorliegen; oder A bedeutet eine Polyarylkette mit 10 bis 14 Kohlenstoffatomen oder Polycyclankette mit 6 bis 10 Kohlenstoffatomen, die durch mindestens eine Alkylengruppe mit 1 bis 8 Kohlenstoffatomen, eine Aminfunktion (-NH-), eine Amidfunktion (-CONR- oder NRCO, worin R eine C₁₋₈-Alkylgruppe bedeutet), eine Etherfunktion (-O-), eine Thioetherfunktion (-S-), ein Wasserstoffatom, eine Carbamatfunktion (-NH(CO)O)- oder -O(CO)NH-), eine Harnstofffunktion (-NH(CO)NH-) unterbrochen sein kann:
n ist eine ganze Zahl im Bereich von 2 bis 5.000 und vorzugsweise 2 bis 200,
oder ein physiologisch akzeptables Salz dieser Verbindungen, und eine Verbindung, die unter den Verbindungen, die das Keratin reduzieren, und den Verbindungen, die das Keratin fixieren, ausgewählt ist.

13. Vorrichtung mit mehreren Abteilungen oder Kit, **dadurch gekennzeichnet, dass** eine Abteilung eine reduzierende kosmetische Zusammensetzung, die eine von einem Polyguanidin abgeleitete Verbindung und eine das Keratin reduzierende Verbindung in Kombination enthält, nach einem der Ansprüche 1 bis 6 enthält.

14. Vorrichtung mit mehreren Abteilungen oder Kit, **dadurch gekennzeichnet, dass** eine Abteilung eine fixierende kosmetische Zusammensetzung, die eine von einem Polyguanidin abgeleitete Verbindung und eine das Keratin fixierende Verbindung in Kombination enthält, nach einem der Ansprüche 1 bis 4 enthält.
